# EUROPEAN PATENT APPLICATION

(11) **EP 1 302 189 A1**
(43) Date of publication of application: **16.04.2003**
(21) Application number: 01949934.2
(22) Date of filing: 11.07.2001
(51) Int. Cl.: A61K 7/00, A61P 17/00

(54) **COSMETIC COMPOSITIONS FOR REDUCING AND METHOD OF USING THE SAME**

(30) Priority: 13.07.2000 JP 2000212689; 04.10.2000 JP 2000304753; 16.03.2001 JP 2001076149
(71) Applicant: Kawai Cosmetic Co., Ltd, Higashiyamato-shi, Tokyo 207-0021 (JP)
(72) Inventor: NAKAMURA, Kazuo, Higashiyamato-shi, Tokyo 207-0021 (JP)
(74) Representative: Giles, Ashley Simon
(86) International application number: JP0106005
(87) International publication number: WO02005755

(57) **Abstract**

In a slimming cosmetic composition for applying a gel cosmetic composition to the skin, then covering the skin surface over the cosmpetic composition and fastening, wherein a lipolytic component is compounded in a gel base. As the lipolytic component, either one or both of anhydrous caffeine and marine algae extract is selected. Butcher's broom extract is compounded in the cosmetic compound. The butcher broom extract has an action of recovering capillary vessels to a normal condition and of removing waste matter with water.

## Description

### TECHNICAL FIELD

The present invention relates to an ideal slimming cosmetic composition for removing fat and extra flesh of each region of the body such as the abdomen, arm, thigh, chin, back and all the like and a method of using thereof.

### BACKGROUND TECHNIQUE

Hitherto, various patents relating to this kind of slimming compositions are acquired. For example, JP-B-60-26083 discloses a slimming composition containing thioester and xanthin derivative.

And, JP-B-3-68004 describes a slenderizing composition containing a plant extract containing saponin, an extract of arnica montanna L and an extract of cola nut.

Moreover, Japanese Patent No. 2656455 describes a slimming composition comprising the first dispersion of a lipid vesicle containing activated medicine selected from fat decomposer, discharger and solidifyer and the second dispersion of a lipid vesicle containing activated medicine slected from skin treatment, trimmer, smoothener and softener.

All conventional slimming compositions try to obtain slimming effect by applying to a skin surface. According to the inventor's study, however, a certain hours are required until components reach subcutaneous fat and decompose, and the hours include time for drying a slimming composition applied to the skin surface. Therefore, the slimming effect of these conventional slimming compositions requires continuous application and massage for at least more than one month. Accordingly, in order to obtain the better effect than the conventional slimming composition, it is found that application to the skin surface is not enough but also a composition having quick permeant function and lipolytic function or any use method having excellent efficiency is necessary.

On the other hand, JP-A-9-295934 describes an aesthetic plaster of the type of applying a slimming composition to a material such as cataplasm, plaster, tape or the like, and plastering on the skin. However, if this aesthetic plaster is not applied through bedtime and continuously used for 30 days, no effect is obtained and definite effect cannot be expected within a short period. Thus, even if the slimming composition is prevented from drying, it becomes clear that such prevention cannot obtain the effect.

The present invention is invented and developed for solving the above problems, proposing a slimming cosmetic composition with permeant function and lipolytic function effected within a short time and obtaining positive effect within a short period by extremely efficiently effecting the slimming cosmetic composition.

### DISCLOSURE OF THE INVENTION

A slimming cosmetic composition of the invention applies a gel cosmetic composition to the skin, then coats and fastens the composition-applied skin surface with a strip coating member, wherein a lipolytic component is compounded with a gel base. The lipolytic component coated and fastened with the strip coating member accelerates permeant force and lipolytic action permeated in the inside of the skin.

As the lipolytic component, one or both of anhydrous caffeine and marine algae extract is selected. Both the anhydrous caffeine and marine algae extract function to decompose fat, where the anhydrous caffeine is excellent in permeant force and the marine algae extract functions to keep the skin from chapping.

The cosmetic composition is compounded in a butcher's broom extract. The butcher's broom extract functions to recover peripheral vessels to a normal condition and to remove waste matter in the body with water.

With the cosmetic composition are compounded any one or all of amino acids such as allantoin, sodium L-aspartate, DL-alanine, L-arginine, lysineh hydrochloride, glycine, L-threonine, L-serine, taurine, L-thyrosin, L-proline, L-isoleucine, L-valine, L-phenylalanine, L-leucine, 5'-disodium inosinic acid, 5'-disodium guanylic acid, L-glutamic acid and L-histidine hydrochloride. The amino acid has moisturizing function for protecting the skin.

The slimming cosmetic composition according to the invention in a method of using the slimming cosmetic composition compounding the lipolytic component in the gel base is to keep still for about 30-50 minutes after warming the cosmetic composition at 40-60°C, applying it to the skin, coating the skin surface with a strip coating member and fastening the member.

The method of using the slimming cosmetic composition according to the invention is, after applying a gel cosmetic composition to the skin, to coat the skin surface with a strip coating member over the cosmetic composition and to fasten it. The lipolytic component coated and fastened with the strip coating member accelerates permeant force and lipolytic action permeated in the body from the skin.

And, a sliming region is warmed with hot water to increase the temperature of the region, the cosmetic composition is applied to the region, the skin surface is coated with a strip coating member over the cosmetic composition and fastened, and thereafter the condition is kept still for about 30-50 minutes. The lipolytic component is permeated into the slimming region warmed in hot water such as a bath to perform lipolytic action.

Further, a steamed towel is pressed on the slimming region for raising the temperature, the cosmetic composition is applied to the skin of the region, the strip coating member is coated and fastened the skin surface over the cosmetic composition, and thereafter the condition is kept for about 30-50 minutes. The lipolytic component is permeated in the slenderizing region warmed with a comparatively easily usable steamed towel to perform lipolytic action.

The other method of using the slimming cosmetic composition according to the invention is, in a method of using the slimming cosmetic composition compounding the lipolytic component in the gel base, to apply the slimming cosmetic composition to the skin after raising the temperature of the slimming region.

Moreover, there is employed means for raising the temperature of the slimming region by means of massage, ultrasonic irradiation and far-infrared irradiation or a combination thereof.

Further, as means for raising the temperature of a slimming region, there is employed means for raising the temperature of the slimming region by pressing a steamed towel on the region.

### BEST MODE FOR CARRYING OUT THE INVENTION

The slimming cosmetic composition and the use method thereof according to the invention are explained in detail. One of fundamental methods of using the slimming cosmetic composition according to the invention is that the cosmetic composition is warmed at 40-60°C and applied to the skin, and thereafter the skin surface is tied and fastened with the strip coating member over the cosmetic composition, and the condition is kept still for about 30-50 minutes.

And, another use method is that the slimming region is warmed, the cosmetic composition applied to the warmed skin, the skin surface is tied and fastened with the strip coating member over the cosmetic composition, and the condition is kept still for about 30-50 minutes.

A further use method is to warm the slimming region and the cosmetic composition is applied to the skin.

As the fundamental component of the optimum slimming cosmetic composition for this method, a lipolytic component is compounded in a gel base. For the lipolytic component, marine algae extract, anhydrous caffeine and the like are preferable. It is also possible to use the other known lipolytic components. Further, at the time of use, in order to protect the skin surface, or to provide moisture, it is preferable to contain amino acid, marronnier extract and water-soluble collagen.. And phenoxyethanol is preferably contained in the cosmetic composition as an antiseptic.

Further, in case of selecting either one or both of anhydrous caffeine and marine algae extract, the anhydrous caffeine has function of accelerating decomposition of body fat. Its permeant force to a depth is excellent, but there is the possibility of irritating the skin. Further, the marine algae extract also has function of accelerating decomposition of body fat. The marine algae extract does not irritate the skin, but has less permeant force. Therefore, selection of either one or both of these anhydrous caffeine and marine algae extract can provide a cosmetic composition suitable for each region of the body.

As the gel base, use is made of a carboxyl vinyl polymer having moisturizing function by dissolving with water. In this case, potassium hydroxide is added as a neutralizer. Further, the other gel base hitherfore used can be used.

Further, to the gel base is added a butcher's broom extract. The butcher's broom extract has the action of recoverig peripheral nerves to the normal condition and removing waste matter in the body. Similarly, tormentila extract can be added. Further, either one or both of a marronnier extract and a soybean extract may be added. The marronnier extract component has function of improving the swelling. Further, the similar effect can be obtained from the soybean extract.

Amino acid particularly has moisturizing function, so as to function for protecting the skin. Amino acid used in the invention contains either one of allantoin, sodium L-aspartate, DL-alanine, L-arginine, lysine hydrochloride, glycine, L-threonine, L-serine, taurine, L-thyrosin, L-proline, L-isoleucine, L-valine, L-phenylalanine, L-leucine, 5'-disodium inosinic acid, 5'-disodium guanylic acid, L-glutamic acid and L-histidine hydrochloride. These amino acids are not limited to a special kind, but contained many kinds little by little as possible so as to be able to obtain stable moisturizing effect suitable to user's age, gender, constitution, etc. Moreover, as the other additives, it becomes possible to prevent damage of the skin at the time of use by using dipotassium glycyrrhizinate added for the purpose of obtaining anti-allergy and anti-inflammation effects, BG (1-3-butylene alcohol), sorbitol solution, trimethylglycine and glucose added for the purpose of obtaining moisturizing effect, water-soluble collagen added as nutriment and gingko extract added as cell activator or the like.

### EXAMPLE

Example 1 shows an example of compounding the following components in a gel base.
- Components contained in the whole quantity of 100g added to refined water

| | |
|---|---|
| BG (1-3-butylene alcohol) | 1.0 (weight %) |
| Sorbitol solution | 1.0 |
| Trehalose | 1.0 |
| Trimethylglycine | 1.0 |
| Phenoxyethanol | 0.5 |
| Anhydrous caffeine | 0.2 |
| Carboxy vinyl polymer | 0.2 |
| Potassium hydroxide | 0.1 |
| Dipotassium glycyrrhizinate | 0.1 |
| Marine algae extract | 0.05 |
| Tormentila extract | 0.05 |
| Soybean extract | 0.05 |
| Glucose | 0.05 |
| Gingko extract | 0.05 |
| Marronnier extract | 0.05 |
| Amino acid | 0.01 |
| Butcher's broom extract | 0.01 |
| Water-soluble collagen | 0.01 |

The amino acid shown in Example 1 includes all of allantoin, sodium L-aspartate, DL-alanine, L-arginine, lysine hydrochloride, glycine, L-threonine, L-serine, taurine, L-thyrosin, L-proline, L-isoleucine, L-valine, L-phenylalanine, L-leucine, 5'-disodium inosinic acid, 5'-disodium guanylic acid, L-glutamic acid and L-histidine hydrochloride.

The components shown in Example 1 are put in a container, and the cosmetic composition of the invention is warmed in boiling water at 60°C. Then, the cosmetic composition of the invention is applied to the portion necessary to be reduced, bleached cotton cloth is wound therearound and fastened, and thereafter the user lies face up and keeps the condition for 40 minutes. The results are shown in the following Tables 1 to 7.

**Table 1**

| | A(male) | B(male) | C(male) | D(female) | E(female) |
|---|---|---|---|---|---|
| region | abdomen | abdomen | double chin | arm | thigh |
| before use(cm) | 100.5 | 95.0 | convex of 0.2 | 31.5 | 39.7 |
| after 1st use | 98.5 | 92.5 | a little | 29.3 | 38.4 |
| after 2nd use | 97.8 | 90.2 | normal | 28.0 | 37.3 |
| after 3rd use | 96.8 | 87.8 | - | 27.4 | 36.7 |
| after 4^{th} use | 95.1 | 84.2 | - | - | - |

Table 1 shows the result of carrying out **4** times by changing respective slimming portion desired by 5 subjects, As the result, effect of 5-10 cm is recognized at the abdominal region. Further, the double chin is tightened with two treatments so as to recover a normal chin. Moreover, tightening effect of 3-4 cm is recognized with respect to the arm and thigh.

In the use method of the invention, it is important to wind and fasten sash-like material such as bleached cotton cloth and the like, but instead of the bleached cotton cloth, it is possible to use bandage and rubber belt, and the quality thereof can freely be selected. Further, a standard of winding the sash-like material is to wind up from the lower part to the upper part of the body. For example, in case of the abdomen, the sash-like material is started to be wound from the lower abdominal region up to the pit of the stomach. In case of the arm, it is wound from the wrist to the armpit or from the elbow to the armpit. In case of the foot, it is wound from the ankle to the lower part of the crotch or from the ankle to the lower part of the knee or from the upper part of the knee to the lower part of the crotch. In case of the jaw and the cheek, it is wound from the lower part of the jaw to overhead. The sash-like material is used for pressing the cosmetic composition to the skin surface with pressure. Upon this, after rubbing in and applying the cosmetic composition of the invention to the skin, the sash-like material is slightly tightly wound. However, sufficient care should be taken for avoiding damage of the blood circulation.

Table 2 to Table 7 show different effects based on each temperature by changing temperatures of the cosmetic composition of the invention.

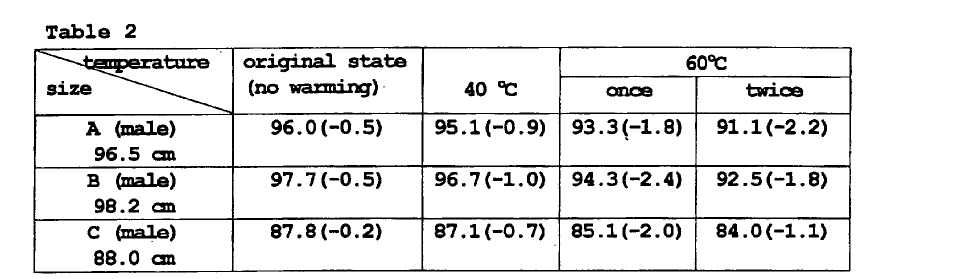

Table 2 shows effect of the abdomen. At the abdomen, the effect of about -0.5 cm is recognized even when the cosmetic composition of the invention is used without warming, but the effect increases from 2 times to 4 times by warming up to 40-60°C. However, effect of using the original solution is limited to the case of fastening with a sash-like material, and when the composition is applied without fastening the sash-like material, no slimming effect is obtained.

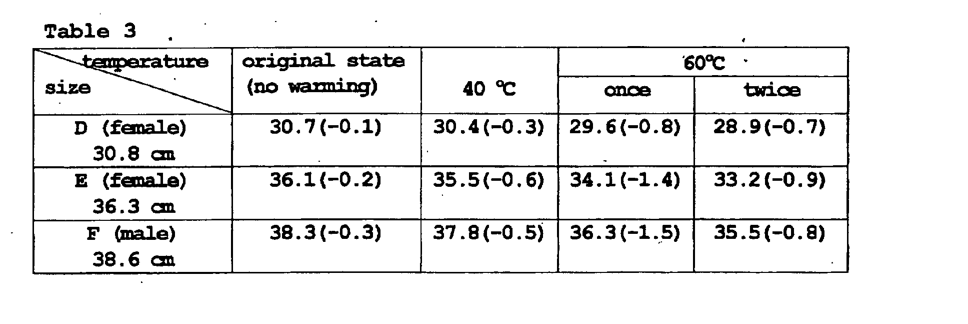

Table 3 shows effect of the arm region. The use at the arm region is not so different between the original solution and 40°C, but when warming to 60°C, difference of effects becomes clear.

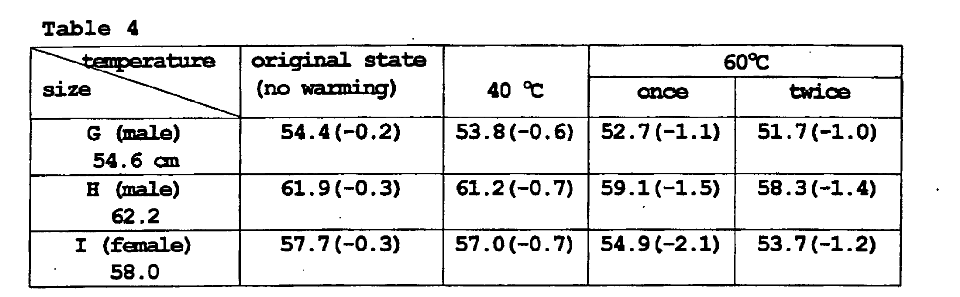

Table 4 shows effect of the thigh region. At the thigh region, the effect becomes remarkable from 40°C and the effect of 2 cm is obtained with only one use at 60°C, so says some subject.

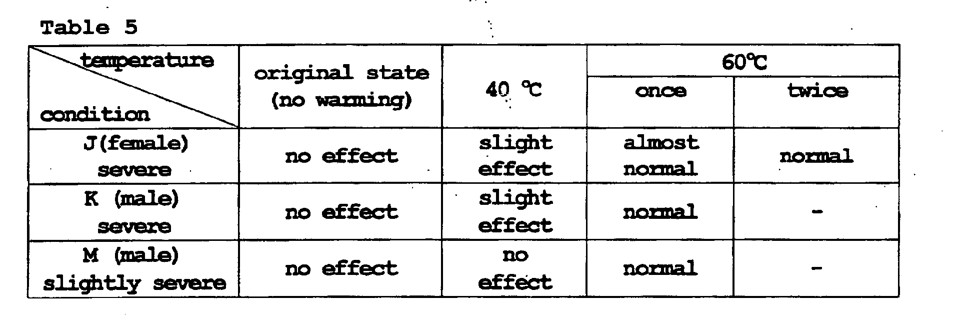

Table 5 shows effect of a double chin and a nape. The double chin and the nape are difficult to measure, and measured with the eye. As a result, no effect is recognized with the original solution, but all the subjects become normal at 60°C.

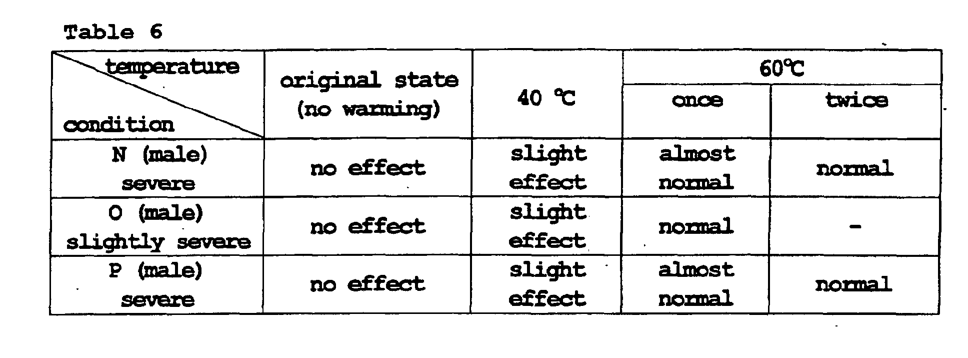

Table 6 shows effect of the buttocks. The effect of the saggy buttocks is judged with the eye. Even in this result, no apparent effect is obtained except at 60°C.

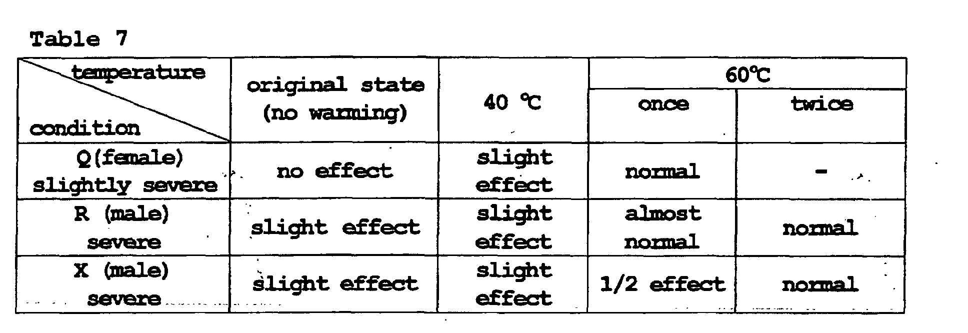

Table 7 shows effect of the back. Extra flesh of the back is also judged with the eye. As a result, it is found that no apparent effect is obtained except at 60°C.

Thus, it is understood that the cosmetic composition of the invention is most effective in case of using at the condition of warming at 60°C.

Next, Example 2 and Example 3 show the method of keeping still under the condition of applying the cosmetic composition after warming the slimming region, winding and fastening the sash-like material around the skin surface over the cosmetic composition for about 30-50 minutes.

### Example 2

The user soaks in a bathtub at about 40°C for 5 minutes for warming the whole body. Then, after a bath, the slimming cosmetic composition of the above component example is applied to the skin, and an expansible bandage (rubber thread is woven in a thick bandage) is wound around the skin surface over the cosmetic composition and fastened. Thereafter, the user lies face up and keeps the condition for 40 minutes, and the result is shown in the following Table 8. More, the columns of the normal temperature show measured values when a slimming cosmetic composition of the normal temperature is applied to the skin, bleached cotton cloth is wound around the skin surface over the slenderizing cosmetic composition and fastened and 40 minutes elapses. Also, the 2nd column is the result carried out by the same method the next day.

**Table 8**

| slimming region | subject | before use | normal temperature (once) | normal temperature (twice) | after use after bathing |
|---|---|---|---|---|---|
| abdomen | A (male) | 108.4 cm | 107.9(-0.5) | no effect | 106.7(-1.2) |
| | B (male) | 96.1 cm | 95.8(-0.3) | no effect | 95.0(-0.8) |
| thigh | C (male) | 47.9 cm | no effect | no effect | 47.4(-0.5) |
| | D(female) | 50.5 cm | 50.3(-0.2) | no effect | 49.6(-0.7) |
| nape | E (male) | severe | no effect | no effect | slight effect |
| | F (female) | severe | no effect | no effect | slight effect |
| double chin | G (male) | moderate | no effect | no effect | slight effect |
| | H (male) | severe | no effect | no effect | slight effect |
| upper arm | I (male) | 30.2 cm | no effect | no effect | 29.8(-0.4) |
| | J(female) | 34.5 cm | no effect | no effect | 34.0(-0.5) |
| saggy buttock | K (female) | severe | no effect | no effect | slight effect |
| | M(female) | moderate | no effect | no effect | slight effect |

It is understood from this result that the most slimming effect is obtained at the abdomen among each region of the body. Further, the case of using the slimming cosmetic composition of the above component example shows slight effect, but almost no effect as compared with the warmed region.

### Example 3

The user makes a steamed towel by firmly wringing the towel warmed with hot water at about 60°C. The steamed towel is applied to a slimming region several times for partially raising the temperature of the slimming region, then the slimming cosmetic composition of the above component example is applied to the skin, and an expansible bandage is wound around the skin surface over the slimming cosmetic composition and fastened. Thereafter, the user lies face up and keeps the condition for 40 minutes. The result is shown in the following Table 9.

**Table 9**

| slimming region | subject | before use | after use with steamed towel |
|---|---|---|---|
| abdomen | A (male) | 106.7 cm | 106.1(-0.6) |
| | B (male) | 95.0 cm | 94.5(-0.5) |
| thigh | C (male) | 47.4 cm | 47.0(-0.4) |
| | D(female) | 49.6 cm | 49.0(-0.6) |
| nape | E (male) | severe | slight effect |
| | F(female) | severe | slight effect |
| double chin | G (male) | moderate | no effect |
| | H (male) | severe | no effect |
| upper arm | I (male) | 29.8 cm | 29.5(-0.3) |
| | J(female) | 34.0 cm | 33.7(-0.3) |
| saggy buttock | K(female) | severe | slight effect |
| | M(female) | moderate | slight effect |

As a result, the temperature of the slimming region is hardly increased as compared with the time after the bath, so that slimming effect is slightly lowered, but slimming effect is recognized at each region except the double chin region. Thus, with the use of the steamed towel, the slimming cosmetic composition can easily be used.

In the use method of the invention, the work of winding and fastening sash-like material such as a bandage and the like is important, but instead of the bandage, it is possible to use bleached cotton cloth, rubber belt and the like can be used, and the quality is freely selected. Further, a standard of winding sash-like material is to be wound from the lower part to the upper part of each region of the body. For example, the abdomen is wound from the underbelly up to the pit of the stomach. The arm is wound from the wrist up to the arm pit or from the elbow to the arm pit. The foot is wound from the ankle to the undercrotch, from the ankle to the lower part of the knee or from the upper part of the knee to the undercrotch. The jaw and cheek are wound from the lower part of the jaw to the upper part of the head. The sash-like material is used for pressing the cosmetic composition to the skin surface with pressure. Therefore, after rubbing the cosmetic composition of the invention in and applying to the skin, the sash-like material is somehow firmly wound. However, sufficient care should be taken for avoiding damage of the blood circulation.

In the other use method of the invention, a method of applying the cosmetic composition to the skin after raising the temperature of the slimming region should apply the slimming cosmetic composition after raising the temperature of the slimming region. As means for raising the bodily temperature of the slimming region, there is employed either one of massage, ultrasonic irradiation and far-infrared irradiation or a combination thereof, or means for raising the bodily temperature of the slimming region by applying a steamed towel to the slenderizing region.

### INDUSTRIAL UTILIZABLILITY

As described above, according to the slimming cosmetic composition and the method of using thereof, permeant action and lipolytic action function within an extremely short time.

## Claims

1. A slimming cosmetic composition for coating the skin surface with a sash-like member over the cosmetic composition and fastening after applying a gel cosmetic composition to the skin, **characterized by** compounding a lipolytic component in a gel base.

2. A slenderizing cosmetic composition as claimed in claim 1, wherein either one or both of anhydrous caffeine and marine algae extract are selected as the lipolytic component.

3. A slimming cosmetic composition as claimed in claim 1 or 2, wherein butcher's broom extract is compounded in the cosmetic composition.

4. A slimming cosmetic composition as claimed in any one of claims 1-3, wherein any one of allantoin, sodium L-aspartate, DL-alanine, L-arginine, lysine hydrochloride, glycine, L-threonine, L-serine, taurine, L-thyrosin, L-proline, L-isoleucine, L-valine, L-phenylalanine, L-leucine, 5'-disodium inosinic acid, 5'-disodium guanylic acid, L-glutamic acid and L-histidine hydrochloride or all amino acids is compounded in the cosmetic composition.

5. A method of using a slimming cosmetic composition compounded a lipolytic component in a gel base, **characterized in that** the cosmetic composition is warmed up to 40-60°C and applied to the skin, then the skin surface is coated and fastened with a sash-like member over the cosmetic composition, and thereafter the condition is kept still for about 30-50 minutes.

6. A method of using a slimming cosmetic composition compounded a lipolytic component in a gel base, **characterized in that**, after applying the slimming cosmetic composition to the skin, the skin surface is coated and fastened with a sash-like member over the cosmetic composition.

7. A method of using a slimming cosmetic composition compounded a lipolytic component in a gel base, **characterized in that** a slimming region is warmed with hot water to raise the bodily temperature of the slimming region, a cosmetic composition is applied to the skin of the slimming region, the skin surface is coated and fastened with a sash-like member over the cosmetic composition, and thereafter the condition is kept still for about 30-50 minutes.

8. A method of using a slimming cosmetic composition compounded a lipolytic component in a gel base, **characterized in that** the bodily temperature of a slimming region is raised by placing a steamed towel on the slimming region, a cosmetic composition is applied to the skin of the slimming region, the skin surface is covered with a sash-like member over the cosmetic composition and fastened, and thereafter the condition is kept still for about 30-50 minutes.

9. A method of using a slimming cosmetic composition compounded a lipolytic component in a gel base, **characterized in that**, after raising the bodily temperature of a slimming region, the slimming cosmetic composition is applied to the skin.

10. A method of using a slimming cosmetic composition as claimed in claim 9, wherein means for raising the bodily temperature of a slimming region is any means of massage, ultrasonic irradiation and far-infrared irradiation or a combination thereof.

11. A method of using a slimming cosmetic composition as claimed in claim 9, wherein means for raising the bodily temperature of the slimming region is a steamed towel placed on the slimming region.
